(19) Europäisches Patentamt
European Patent Office
Office européen des brevets

(11) Veröffentlichungsnummer: **0 260 585 B1**

(12) # EUROPÄISCHE PATENTSCHRIFT

(45) Veröffentlichungstag der Patentschrift: **16.10.91**

(21) Anmeldenummer: **87113152.0**

(22) Anmeldetag: **09.09.87**

(51) Int. Cl.5: **C07C 49/633**, C07C 49/533, C07C 49/443, C07C 49/323, C07C 13/44

(54) **Bicyclische Ketone, Verfahren zu deren Herstellung und Riech- und/oder Geschmackstoffkompositionen mit einem Gehalt an diesen bicyclischen Ketonen.**

(30) Priorität: **18.09.86 CH 3740/86**

(43) Veröffentlichungstag der Anmeldung:
**23.03.88 Patentblatt 88/12**

(45) Bekanntmachung des Hinweises auf die Patenterteilung:
**16.10.91 Patentblatt 91/42**

(84) Benannte Vertragsstaaten:
**CH DE FR GB LI NL**

(56) Entgegenhaltungen:
**EP-A- 0 116 277**
**DE-A- 2 440 406**
**GB-A- 1 300 970**

**TETRAHEDRON LETTERS, Band 24, Nr. 42, 1983, Seiten 4595 - 4598, Verbindung 9, Oxford, GB; G.W. KLUMPP et al.: "Diradicals in the vinylcyclopropane rearrangement of bicyclo (3.1.0) hexane derivatives"**

**TETRAHEDRON 30, 2267 - 2271 (1974)**

(73) Patentinhaber: **L. GIVAUDAN & CIE Société Anonyme**

**CH-1214 Vernier-Genève(CH)**

(72) Erfinder: **Naegeli, Peter, Dr.**
**Vordere Höhenstrasse 31**
**CH-5430 Wettingen(CH)**

(74) Vertreter: **Urech, Peter, Dr. et al**
**Grenzacherstrasse 124 Postfach 3255**
**CH-4002 Basel(CH)**

**EP 0 260 585 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Riech- und/oder Geschmackstoffe. Es handelt sich dabei um die Verbindungen der allgemeinen Formel

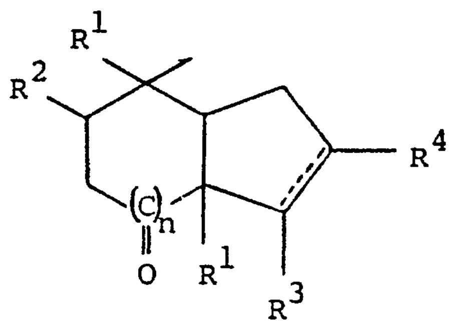

I

worin $R^1$ Methyl oder Aethyl, $R^2$ Wasserstoff oder Methyl, und falls n = 0, $R^3$ = Wasserstoff und $R^4$ Acetyl oder Propionyl, und, falls n = 1, $R^3$ = Methyl oder Aethyl und $R^4$ Wasserstoff oder Methyl darstellen, und die gestrichelte Linie eine fakultative C-C-Bindung darstellt,
mit der Ausnahme von 1,4,4,7a-Tetramethyl-3a,4,5,7a-tetrahydro-7(6H)-ind-1-enon (Ia), also der Verbindung der Formel

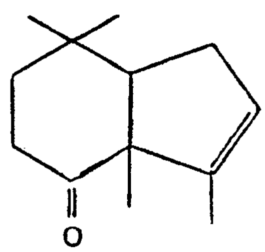

Ia

Diese Verbindung Ia ist aus Tetrahedron 30, 2267 - 2271 (1974) - ohne irgendwelche Hinweise auf organoleptische Eigenschaften - bekannt geworden; siehe daselbst die Verbindung 14.

Die Formel I soll sämtliche möglichen Stereoisomeren umfassen, die sich durch die verschiedenen asymmetrischen Zentren dieser Moleküle ergeben.

Die Verbindungen I weisen besondere organoleptische Eigenschaften auf, auf Grund derer sie sich vorzüglich als Riech- und/oder Geschmackstoffe eignen.

Die Erfindung betrifft demgemäss auch die Verwendung der Verbindungen I als Riech- und/oder Geschmackstoffe und Riech- und/oder Geschmackstoffkompositionen mit einem Gehalt an Verbindungen I.

Die Erfindung betrifft auch ein Verfahren zu deren Herstellung. Dieses Verfahren ist dadurch gekennzeichnet, dass man eine Verbindung der Formeln

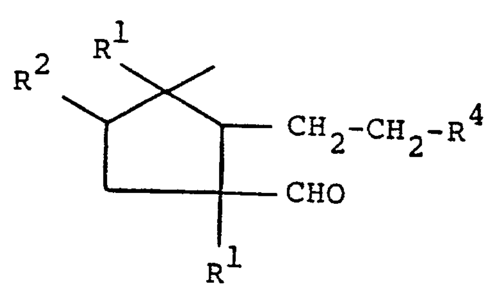

II

worin $R^1$ und $R^2$ obige Bedeutungen besitzen und $R^4$ Acetyl oder Propionyl bedeutet,

oder

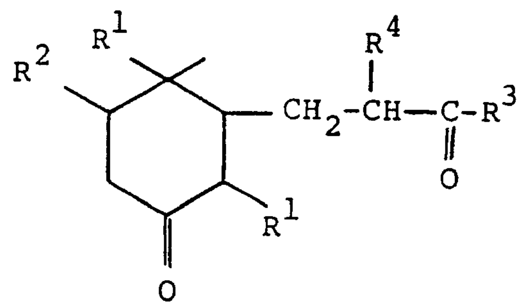

III

worin $R^1$ und $R^2$ obige Bedeutungen besitzen, $R^3$ Methyl oder Aethyl und $R^4$ Wasserstoff oder Methyl darstellen,

cyclisiert und, gegebenenfalls, die C = C-Doppelbindung des erhaltenen Reaktionsproduktes hydriert.

Die Cyclisierung der Verbindung II erfolgt zweckmässigerweise mittels Basen, z.B. Alkalimetall- oder Erdalkalimetallhydroxden oder Alkalimetall- oder Erdalkalimetallcarbonaten. Man arbeitet zweckmässigerweise in einem Lösungsmittel, wie einem Kohlenwasserstoff oder einem Alkohol oder Aether, und bei Zimmertemperatur, oder, vorzugsweise, bei erhöhter Temperatur.

Die Cyclisierung der Verbindung III erfolgt zweckmässigerweise mittels Protonsäuren, z.B. mittels Mineralsäuren wie Salzsäure, Schwefelsäure, Phosphorsäure, Perchlorsäure, etc. oder mittels organischen Sulfonsäuren wie p-Toluolsulfonsäure. Die zweckmässigen Reaktionsbedingungen sind analog.

Die Hydrierung des primär gebildeten Reaktionsproduktes erfolgt zweckmässigerweise katalytisch, z.B. mittels Edelmetallen auf Trägern, z.B. Pd[C]. Die Anwesenheit eines Lösungsmittels ist fakultativ, geeignete Lösungsmittel sind Alkohole, Ester, Kohlenwasserstoffe.

Die Verbindungen der Formeln II und III werden vorzugsweise in situ hergestellt, nämlich aus Verbindungen der Formel

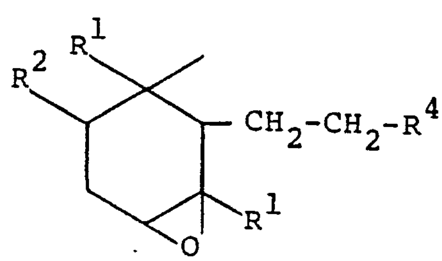

IV

unter dem Einfluss üblicher Lewissäuren, bzw. aus den Verbindungen

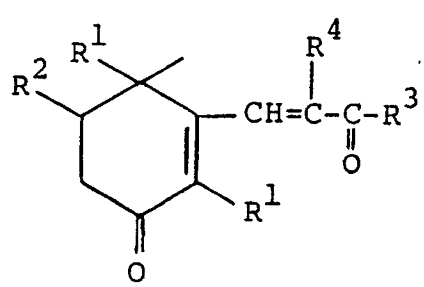

V

durch katalytische Hydrierung. Als Lewissäuren geeignet sind beispielsweise $SnCl_4$, $TiCl_4$, oder, vorzugsweise, $BF_3$ (ätherat). Geeignete Lösungsmittel sind: (chlorierte) Aliphaten und Aromaten und Aether, vorzugsweise Methylenchlorid. Die Reaktionsbedinungen sind zweckmässigerweise mild, z.B. ist ein Temperaturbereich von ca. -20° C bis ca. +50° C zweckmässig.

Zweckmässige Katalysatoren sind Palladium und Platin, mit oder ohne Träger. Bervorzugt ist Pd(C).

Man arbeitet zweckmässigerweise unter dem Einfluss von Basen und in aliphatischen Lösungsmitteln, oder in Alkoholen.

Die erfindungsgemäss als Reich- und/oder Geschmackstoffe verwendeten Verbindungen der Formel I zeichnen sich durch angenehme, ausgesprochen näturlich wirkende Eukalyptusnoten aus; sie sind kräftig und frisch-camphrig-borneolartig, ohne die Flüchtigkeit der Eukalyptusöl-Kopfnoten zu besitzen. Die auftretenden Nebennoten sind: (leicht)fruchtig, tabakartig, cedrig, wurzelig, patchouliartig, pinienartig. Zudem zeichnen sie sich durch ein hohes Diffusionsvermögen, verbunden mit einer ungewöhnlichen Haftfestigkeit aus. Bereits in geringen Konzentrationen eingesetzt, verstärken und veredeln sie das Geruchsbild von Riechstoffkompositionen, insbesondere von fougèreartigen, chypreartigen, holzigen, animalischen Kompositionen oder Basen. Die Verbindungen I verbinden sich mit zahlreichen bekannten Riechstoffingredienzien natürlichen oder synthetischen Ursprungs, wobei die Palette der natürlichen Rohstoffe sowohl leicht- als auch mittel-und schwerflüchtige Komponenten, und diejenige der Synthetika Vertreter aus praktisch allen Stoffklassen umfassen kann, wie dies aus der folgenden Zusammenstellung ersichtlich ist:

- Naturprodukte: Basilikumöl, Baummoos Absolue, Beifussöl, Bergamotteöl, Cassisknospen Absolue, Castoreum, Cedernholzöl, Ciste Labdanum, Civette, Corianderöl, Eichenmoos, Elemiöl, Fichtennadelöl, Galbanum, Geraniumöl, Gewürnelkenöl, Jasmin Absolue und sein synthetischer Ersatz, Jonquille Absolue, Kamillenöl, Labdanum, Lavendelöl, Mandarinenöl, Mastix Absolue, Mentha citrata-öl, Myrrhenöl, Palmarosaöl, Patchouliöl, Petitgrainöl Paraguay, Sandelholzöl, Thymianöl, Vassouraöl, Moschus Infusion, Styrax, Birkenteer, Vetiveröl, Weihrauch, Ylang-Ylang-Oel, Zitronenöl, Zibetöl, etc.
- Alkohole: Citronellol, Dimetol®(2,6-Dimethyl-heptan--2-ol), Geraniol, cis-3-Hexenol, Linalool, Nonadyl®-(6,8-Dimethyl-nonan-2-ol),Phenyläthylalkohol, Rhodinol, Sandela®(3-Isocamphyl-5-cyclohexanol), Sandalore®(3-Methyl--5-(2',2',3'-trimethyl-cyclopenta -3'-en-1'-yl)-pentan-2-ol), Terpineol, etc.
- Aldehyde: α-Amylzimtaldehyd, Cyclamenaldehyd, Decanal, Dodecanal, Heliotropin, α-Hexylzimtaldehyd, Hydroxycitronellal, Lyral, Adoxal®(2,6,10-Trimethyl-9-en-1-al), Undecanal, ω-Undecylenaldehyd, Vanillin, etc.
- Ketone: Isoraldeine®(Isomethyl-α-jonon), α-Jonon, β-Jonon, 3-Prenylisocaranon, Vertofix®-(=acetyliertes Cedernholzöl), p-Methylacetophenon, etc.
- Ester: Acetessigsäureäthylester, Amylsalicylat, Benzylacetat, Cedrylacetat, Cinnamylformiat, Citronellylacetat, Geranylacetat, cis-3-Hexenylacetat, cis-3-Hexenylbenzoat, Linalylacetat, Linalylanthranilat, Methyldihydrojasmonat, Methambrat®(1-Acetoxy-1-methyl-2-sec.-butylcyclohexan), Myraldylacetat®-(4-(4-Methyl-3-pentenyl)-cyclohex-3-en -1-yl-carbinylacetat), Phenoxyäthylisobutyrat, Phenyläthyltiglat, Styrallylacetat, Terpenylacetat, 2,3,6,6-Tetramethylcyclohex-2-en-carbonsäure-äthylester, 3,6,6-Trimethyl-2-äthyl-cyclohex-2-en-carbonsäure-äthylester, Vetivenylacetat, ortho-tert.Butylcyclohexylacetat, etc.
- Verschiedene: Ambrettemoschus, Cumarin, Epoxycedren, Eugenol, Fixolide®(1,1,2,4,4,7-Hexamethyl-6-acetyl -1,2,3,4-tetrahydronaphthalin), Galaxolid®(1,3,4,6,7,8-Hexahydro-4,6,6,7,8,8-hexamethyl -cyclopenta-γ-2-benzopyran), Heliotropin, Indol, Indolen, Isoeugenol, Isobutylchinolin, Jasmonyl(1,3-Diacetoxy-nonan), Ketonmoschus, Limonen, p-Menthan-8-thiol-3-on, Madrox®(1-Methylcyclododecylmethyläther), Methyleugenol, Musk 174® (12-Oxahexadecanolid), γ-Nonalacton, γ-Undecalacton, etc.

Die Verbindungen der Formel I (bzw. deren Gemische) lassen sich in weiten Grenzen einsetzen, die beispielsweise von 0,1 (Detergentien) - 30% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,1 und 25%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I (bzw. deren Gemische) können demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben angeführten bekannten Riechstoffe bzw. Riechstoffgemische nach (dem Parfümeur bekannter) Art und Weise verwendet werden, wie z.B. aus W.A. Poucher, Perfumes, Cosmetics, Soaps 2, 7. Auflage, Chapman und Hall, London 1974 hervorgehend.

Die neuen Verbindungen der Formel I sind ebenfalls vorzüglich geeignet zur Verwendung in Aromen verschiedenster Art, insbesondere aber auch zur Aromatisierung von Tabak.

Als Geschmackstoffe können die Verbindungen I beispielsweise zur erzeugung bzw. Verbesserung, Verstärkung, Steigerung oder Modifizierung von Fruchtaromen, z.B. Heidelbeer-oder Brombeeraromen verwendet werden. Als Anwendungsgebiet dieser Aromen kommen beispielsweise Nahrungsmittel (Joghurt,

Süsswaren etc.), Genussmittel (Tee, Tabak, etc.) und Getränke (Limonade etc.) in Frage.

Die ausgeprägten geschmacklichen Qualitäten der Verbindungen I ermöglichen die Verwendung als Aromastoffe in geringen Konzentrationen. Eine geeignete Dosierung umfasst beispielsweise den Bereich von 0,01 ppm - 100 ppm, vorzugsweise den Bereich von 0,01 ppm - 20 ppm im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 30-100 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1-10, insbesondere 0,5-3 Gew.%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammnstellung bereits enthalten oder können leicht der Literatur entnommen werden, wie z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser, Aethanol, Propylenglykol, Glycerin, etc.

<u>Beispiel 1</u>

a) 60 g 3-[3-Oxobutyl]-2,4,4-trimethyl-cyclohexanon (Produkt der katalytischen Hydrierung von 4-Ketoionen mit Palladium-Kohle in Essigester bei Raumtemperatur und Normaldruck, siehe H. Jwamuro et al., $23^{th}$ Proceedings of the Chemical Society of Japan, 86 [1979]) werden in 500 ml Diäthyläther gelöst vorgelegt und bei Rückflusstemperatur der Lösung sorgfältig unter Rühren 25 ml 80%ige Schwefelsäure zugetropft. Noch viermal werden nach je 1 Stunde Reaktionszeit weitere 25 ml 80%ige Schwefelsäure zugetropft. 30 Minuten nach der letzten Zugabe wird das bräunliche Reaktionsgemisch auf Eis gegossen, mit Wasser auf ca. 1,5 l verdünnt, mit Ammonsulfat die Emulsion gebrochen und die Wasserphase abgetrennt. Nach Waschen mit Natriumbicarbonatlösung und Wasser wird die organsiche Phase wie üblich getrocknet und unter Vakuum eingedampft. Nach Flachdestillation bei 55-60 °C/0,05 Torr erhält man 46 g 1,4,4,7a-Tetramethyl-3a,4,5,7a-tetrahydro-7(6H)-ind-1-enon.

Geruch: an Trockenfrüchte erinnernd, camphrig-cineolig, aber haftfester, holzig-terpenig, an Honig erinnernd, leicht patchouliartig.

| | |
|---|---|
| MS: m/e = | 192(40), 177(35), 159(5), 149(6), 135(32), 123(28), 121(27), 107(27), 99-(31), 95(100), 79(55), |
| IR (Film): | 1700, 1470, 1445, 1375, 1235, 1150, 1080, 1025, 835 $cm^{-1}$. |
| NMR (400 MHz in $CDCl_3$): | $\delta$=4,47 ppm (1H, schmales m); $\delta$ = 2,125 ppm (1H,dxdd,J=2/7/8 Hz), $\delta$ = 1,99 ppm (1H,dxdd,J=7/9/14 Hz); $\delta$ = 1,55 ppm (3H, schmales m); $\delta$ = 1,25 ppm (3H,s); $\delta$ = 1,00 ppm (3H,s); $\delta$ = 0,924 ppm (3H,s). |

b) Alternativvariante: Man löst das Edukt in der 6-7-fachen Gewichtsmenge Eisessig, gibt kalt 1/5 - 1/7 des Volumens an 70%iger Perchlorsäure zu und lässt die Lösung 4 Stunden bei Raumtemperatur rühren. Die Aufarbeitung erfolgt analog wie oben.

c) Die Cyclisationsreaktion des Diketons kann schliesslich analog mit Hilfe von p-Toluolsulfonsäure in siedender Toluollösung durchgeführt werden.

<u>Beispiel 2</u>

1,92 g des obigen bicyclischen Ketons I werden gelöst in 200 ml Hexan mit 1 g 5% Palladiumkohle als Katalysator unter Wasserstoffatmosphäre bei Raumtemperatur hydriert. Nach Filtration über Celite wird eingedampft und das Produkt flach destilliert.

Sdp. des 1,4,4,7a-Tetramethyl-3a,4,5,7a-tetrahydro-7-(6H)-indanons: ~80° C/0,03 Torr.
Geruch: frisch, kräftig, gut haftend, leicht holzig.

| | |
|---|---|
| MS: m/e = | 194(10), 179(40), 161(7), 152(18), 139(100), 123(58), 109(46), 95(72), 81(45) |
| IR (Film): | 1695, 1460-1480, 1375-1380, 1250, 1140, 1120, 1040, 920 $cm^{-1}$ |
| NMR (400 MHz, $CDCl_3$): | Hauptisomer zeigt δ = 2,455 ppm (1H),dxt,J=9/19 Hz); δ = 2,15 ppm (1H,dxdd, J=19/9/3 Hz); 1,235 ppm (3H,s); δ = 0,98 ppm (3H,s); δ = 0,92 ppm (3H,s); δ = 0,90 ppm (3H,d,J=7 Hz) |

Beispiel 3

Das bevorzugte Verfahren zur Herstellung der Verbindungen I (n=1) wird anhand zweier typischer Vertreter exemplifiziert, wobei die der Cyclisation zu unterwerfenden Edukte aus den bekannten, doppelt ungesättigten Diketonen erhalten werden. Deren Weiterverarbeitung kann ohne Isolation und/oder Reinigung erfolgen.

A. 1-Aethyl-4,4,7a-trimethyl-3a,4,5,7a-tetrahydro-7(6H)-ind-1-enon:

95 g 2,4,4-Trimethyl-3-(3-oxo-1-pentenyl)-2-cyclohexen-1-on werden in 1,5 l Hydrieralkohol gelöst, 34 g Kaliumhydroxydplätzchen zugegeben und schliesslich 10 g 5% Palladiumkohle. Darauf wird unter Wasserstoffatmosphäre bis zum Ende der Gasaufnahme (ca. 15 Stunden) intensiv gerührt. Nach Filtration vom Katalysator über Celite wird die Lösung im Vakuum konzentriert, mit Wasser verdünnt und mit Hexan extrahiert. Nach Trocknung und Entfernung des Lösungsmittels verbleiben 90 g viskoses Oel, welches langsam kristallisiert. Die Analyse des Produkts zeigt, dass neben dem gesättigten monocyclischen Diketon III ein grösserer Anteil überbrücktes bicyclisches Aldol gebildet wird. Diese Tatsache hat keinen Einfluss auf die nachfolgende Cyclisationsreaktion, da sich dieses Aldol unter den Cyclisationsbedingungen wieder zu III öffnet und im gewünschten Sinn zu I reagiert. Das obige Rohprodukt III wird hierauf in 445 ml Eisessig und 89 ml 70% Perchlorsäure kalt gelöst und während 5 Stunden bei Raumtemperatur gerührt und dann auf Eis/Wasser (im Ueberschuss) gegossen und mit Hexan erschöpfend extrahiert. Nach Neutralwaschen und Trocknen sowie Eindampfen des organischen Lösungsmittels wird das Rohprodukt I an einer Füllkörperkolonne fraktioniert destilliert, nachdem es an einer kurzen Vigreuxkolonne bei 83-102° C/0,05 Torr von den hochsiedenden Rückständen durch Flachdestillation befreit worden ist.

B. 1,2,4,4,7a-Pentamethyl-3a,4,5,7a-tetrahydro-7(6H)-ind-1-enon:

Das als Ausgangsmaterial eingesetzte 2-(2-Methyl-3-oxobut-1-enyl)-1,3,3-trimethyl-cyclohexen wird durch Isomerisieren von 3-(2-Methyl-3-oxobut-1-enyl)-2,4,4-trimethyl-cyclohexen mit einem 1:1-Gemisch von konz. Schwefelsäure und Aether oder Dichlormethan bei Raumtemperatur innert 16 Stunden erhalten. Es wird mit Wasser neutral gewaschen, getrocknet und unter Vakuum flachdestilliert. 123 g dieses Produkts werden unter Rückflusstemperatur zu einer Lösung von 99 g N-Hydroxyphthalimid in 2,1, l Aceton getropft, darauf werden 1,7 g Dibenzoylperoxyd zugegeben und die Lösung mit Hilfe eines Begasungsrührers in einem Sauerstoffstrom von 150 ml/Min. 24 Stunden reagieren gelassen. Das Reaktionsgemisch wird sorgfältig eingedampft und der Rückstand bei 50° C in 1,2 l Tetrachlormethan aufgenommen, darauf am Rotationsverdampfer zur besseren Kristallisation bei 15° C rotiert, und vom Kristallbrei filtriert (Nachwaschen des Filterkuchens mit $CCl_4$). Das Filtrat wird sorgfältig und unter Kühlung mit einer Lösung aus 200 ml Pyridin und 70 ml Acetanhydrid versetzt, wobei die Erwärmung verzögert einsetzt. Die Lösung wird 12 Stunden bei Raumtemperatur nachgerührt, dann einge- dampft. Die 179 g Rohprodukt V werden in einer Hexan-Aetherlösung (4:1) über 270 g neutralem Aluminiumoxid (Aktivität II) filtriert. Das eingedampfte Eluat wird mit 60 g Paraffinöl versetzt und bei 0,06 Torr/125° C destilliert. Falls die nachfolgende katalytische Hydrierung des Produkts mit 10% Palladiumkohle nicht spontan anläuft, muss das Destillat nochmals in Aether aufgenommen, mit 10% Ferrosulfatlösung verrührt und nach üblicher Aufarbeitung erneut destilliert werden. Die Ausbeute an 3-(2-methyl-3-oxobut-1-enyl)-2,4,4-trimethyl-cyclohex-2-enon beträgt 63%.

| | |
|---|---|
| MS: m/e = | 220(23), 205(5), 191(9), 177(50), 164(23), 149(16), 136(30), 123(10), 107(10), 91(12), 77(20) |
| IR (Film): | 1670, 1600, 1365, 1350, 1330, 1300, 1250, 1200, 1150, 1100, 1030, 995, 885 $cm^{-1}$ |
| NMR (400 MHz, $CDCl_3$). | δ = 7,1 ppm (1H,schmales m); δ = 2,55 ppm (2H, dd, J=6/7 Hz); δ = 2,415 ppm (3H, s); δ = 1,925 ppm (2H, breites t, J= 7/6 Hz); δ = 1,69 und 1,625 ppm (je 3H, schmalstes d, J= 1 Hz); δ = 1,18 ppm (6H, s) |

UV (AetOH): $\lambda_{max}$ = 258 nm ($\epsilon$ = 11600).

77 g des obigen Zwischenproduktes werden in 150 ml absolutem Alkohol gelöst, vom Niederschlag abfiltriert und das Filtrat mit 1,7 l absolutem Alkohol verdünnt. Dazu gibt man 35 g 85% Kaliumhydroxyd und 14,8 g 10% vorhydrierte Palladiumkohle, und rührt unter Wasserstoffatmosphäre bis zum Ende der Wasserstoffaufnahme. Nach Filtration unter Schutzgas wird die Lösung eingeengt, das Produkt in Wasser aufgenommen und mit Hexan erschöpfend extrahiert. Nach Neutralwaschen der organischen Phase wird getrocknet und eingedampft. Aus den 65 g öligen Rohprodukts III lassen sich aus der Lösung in wenig Hexan durch Abkühlen ca. 15 g des bicyclischen anellierten Aldols gewinnen (Smp. ca. 84° C). Für die Herstellung der Verbindung I kann das Rohprodukt III der Hydrierung direkt in 150 ml Eisessig plus 30 ml 70%iger Perchlorsäure gelöst werden. Nach Rühren bei Raumtemperatur (16 Stunden) wird mit Hexan erschöpfend extrahiert und mit Wasser und Natriumbicarbonat neutralgewaschen, getrocknet und eingedampft. Das ölige Rohprodukt (~45 g) wird kurzwegdestilliert bzw. fraktioniert destilliert und ergibt 26 g Reinprodukt I.

Physikalische Daten für die so hergestellten Produkte III (n = 1) und I (n = 1):

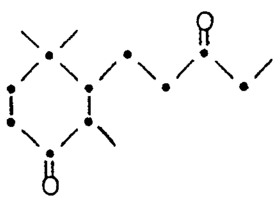

Vgl. Beispiel 3B.

| | |
|---|---|
| Sdp.: | 120° C/0,05 Torr |
| MS: m/e = | 224(1), 206(2), 195(4), 191(2), 177(6), 168(5), 163(2), 152(100), 139(50), 125(28), 111(50), 97(25), 81(18), 69(39), 57(98), 43(84) |
| IR (Film): | 1710, 1470, 1460, 1415, 1390, 1370, 1350, 1155, 1115, 1010 $cm^{-1}$ |
| NMR (400 MHz, $CDCl_3$) Hauptisomer: | $\delta$ = 1,07 ppm (3H,s); $\delta$ = 1,06 ppm (3H,t,J = 7 Hz); $\delta$ = 1,05 ppm (3H,d,J = 6 Hz); $\delta$ = 0,99 ppm (3H, s). |

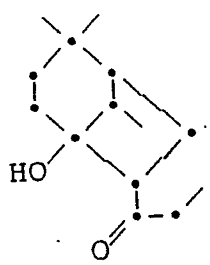

"überbrücktes bicyclisches Aldol"

Vgl. Beispiel 3A

| | |
|---|---|
| MS m/e: | 224(8), 206(3), 195(10), 177(4), 168(3), 153(51), 139(100), 125(21), 107(9), 97(27), 84(69), 69(26), 57(82), 43(40) |
| IR ($CDCl_3$) | : 3550, 1700, 1475, 1460, 1390, 1380, 1370, 1350, 1180, 1140, 1080, 950, 9210 $cm^{-1}$ |
| NMR (400 MHz, $CDCl_3$): | $\delta$ = 2,99 ppm (1H,dd,J = 12/6 Hz); $\delta$ = 2.73 ppm (1H,dq,J = 18/7 Hz); $\delta$ = 2,46 ppm (1H,dq,J = 18/7 Hz); $\delta$ = 2,18 ppm (1H,dd,J = 14/6 Hz); $\delta$ = 2,05 ppm (1H,q,J = 7 Hz); $\delta$ = 1,43 ppm (1H,dxdd,J = 13/6 Hz); $\delta$ = 1,38 ppm |

(1H,br.d,J=7 Hz); δ = 1,1 ppm (1H,dxm,J=14 Hz); δ = 1,055 ppm (3H,t,J=7 Hz); δ = 1,01 ppm (3H,d,J=7 Hz); δ = 0,965 u. 0,90 ppm (je 3H,s).

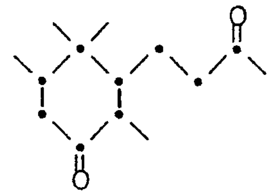

Analog Beispiel 3A.

| | |
|---|---|
| Hauptisomer: | |
| MS m/e: | 224, 209, 191 (alle Spuren), 166(25), 153/154(5-6), 137/139(2-3), 123(7), 111(22-25), 97(9-12), 83(15), 69(30-35), 55(33), 43(100) |
| IR (Film): | 1710, 1460, 1425, 1380-1360, 1285, 1190, 1170, 1080, 1040, 995 $cm^{-1}$ |
| NMR (400 MHz, $CDCl_3$): | δ = 2,15 ppm (3H,s); δ = 2,10 ppm (1H,dxdd,J=14/4/2 Hz); δ = 1,96 ppm (1H,m); δ = 1,08 ppm (3H,d,J=7 Hz); δ = 1,01 ppm (3H,s); δ = 0,965 ppm (3H,d,J=6 Hz); δ = 0,92 ppm (3H,s). |
| Nebenisomer: | Smp. 40° C |
| MS: | wie oben |
| IR (KBr): | 1700, 1470, 1455, 1430, 1410, 1400, 1375/1360, 1340, 1275, 1245, 1230, 1175, 1170, 1050, 970, 995, 925, 890, 805 $cm^{-1}$ |
| NMR (400 MHz, $CDCl_3$): | δ = 2,545 ppm (1H,dxdd,J=17/11/5 Hz); δ = 2,42 ppm (1H,dxdd,J=17/11/5 Hz); δ = 2,145 ppm (3H,s); δ = 1,91 ppm (1H,m); δ = 1,645 ppm (1H,m); δ = 1,585 ppm (3H,s); δ = 1,465 ppm (1H,m); δ = 1,05 ppm (3H,d,J=6 Hz); δ = 0,995 ppm (1H,dxdd); δ = 0,965 ppm (3H,s); δ = 0,94 ppm (3H,d,J=6 Hz); δ = 0,90 ppm (3H,s). |

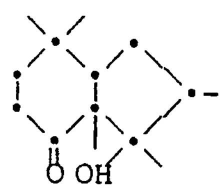

"anelliertes bicyclisches Aldol"

Vgl. Beispiel 3B.

| | |
|---|---|
| MS: m/e = | 224(1), 209(1), 206(8), 191(4), 179(1), 168(3), 163(5), 152(100), 149(13), 139-(20), 125(49), 109(19), 93(12), 81(8), 72(18), 69(20), 55(28), 43(86) |
| IR ($CDCl_3$): | 3600, 3530, 1680, 1460, 1380, 1155, 1012, 925, 890 $cm^{-1}$ |
| NMR (400 MHz, $CDCl_3$): | δ = 1,22 ppm (3H,s); δ = 1,12 ppm (3H,s); δ = 0,965 ppm (3H,s); δ = 0,94 ppm (3H,d,J=6 Hz); δ = 0,84 ppm (3H,s). |

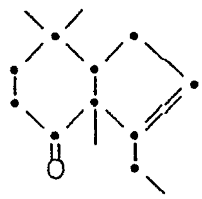

Vgl. Beispiel 3A.

| | |
|---|---|
| Sdp. | 90° C/=0,02 Torr |
| Geruch: | sehr kräftig, camphrig, holzig, cedrig, an Iron erinnernd, Patchouli, Eukalyptus, tabakig, langhaftend. |
| MS: m/e = | 206(31), 191(12), 177(43), 163(13), 149(40), 135(20), 121(29), 109(100), 99-(24), 93(97), 81(66), 69/67(31-33), 55(40), 43(73) |
| IR (Film): | 1700, 1460, 1420, 1390, 1370, 1315, 1270, 1235, 1190, 1140, 1120, 1090, 1060, 1030, 1010, 885, 830 $cm^{-1}$ |
| NMR (440 MHz, $CDCl_3$): | $\delta$ = 5,48 ppm (1H,q, schmal); $\delta$ = 2,265 ppm (1H,dxdd,J=17/9/7 Hz); $\delta$ = 2,125 ppm (1H,dxdd,J= 10/6/2 Hz); $\delta$ = 1,995 ppm (1H,dxdd,J=17/9/7); $\delta$ = 1,245 ppm (3H,s); $\delta$ = 1,01 ppm (3H,t,J=7 Hz); $\delta$ = 0,995 ppm (3H,s); $\delta$ = 0,93 ppm (3H,s). |

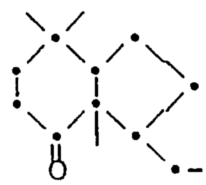

Analog Beispiel 2.

| | |
|---|---|
| Sdp.: | 60° C/0,02 Torr |
| Geruch: | Frisch, nach Cineol, camphrig, süss, stark und homogen, holzig |
| MS: m/e = | 208(14), 193(31), 179(12), 165(10), 152(50), 139(100), 123(31), 109(62), 97-(37), 81(35), 69(24), 55(39), 43(45), 28(45) |
| IR (Film): | 1692, 1460, 1410, 1385, 1370, 1240, 1130, 1110, 1025, 1000, 945, 925, 885 $cm^{-1}$ |
| NMR (400 MHz, $CDCl_3$): | Isomer A: $\delta$ = 1,25 ppm (3H,s); $\delta$ = 0,97 ppm (3H,s); $\delta$ = 0,92 ppm (3H,s); $\delta$ = 0,85 ppm (3H,t,J=7 Hz). Isomer B: $\delta$ = 0,99 ppm (3H,s); $\delta$ = 0,925 ppm (3H,s); $\delta$ = 0,875 ppm (3H,t,J=7 Hz); $\delta$ = 0,83 ppm (3H,s). |

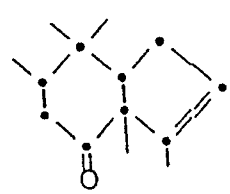

Analog Beispiel 3A.

| | |
|---|---|
| Geruch: | stark, haftend, iononartig, camphrig, holzig |
| MS: m/e = | 206(23), 191(28), 177/173(2), 163(4), 149(2), 135(47), 95(100), 91(23), 79- |

| | |
|---|---|
| | (56), 69(27), 55(26), 41(53), |
| IR (Film): | 3040, 1700, 1450, 1412, 1392, 1370, 1320, 1285, 1235, 1190, 1092, 1030, 945, 830/810/792 $cm^{-1}$ |
| NMR (400 MHz, $CDCl_3$): | $\delta$ = 5,335 ppm (1H,m schmal); $\delta$ = 2,43 ppm (1H,dd,J=18/6 Hz); $\delta$ = 2,255 ppm (1H, breites d,J=17 Hz); $\delta$ = 2,07 ppm (1H,dd,J= 8,5/3 Hz); $\delta$ = 2,04 ppm (1H,dd,J= 17/12 Hz); $\delta$ = 1,92 ppm (1H, pseudo septett.); $\delta$ = 1,71 ppm (3H, schmales m); $\delta$ = 1,225 ppm (3H,s); $\delta$ = 0,925 ppm (3H,d,J=7 Hz); $\delta$ = 0,92 ppm (3H,s); $\delta$ = 0,655 ppm (3H,s). |

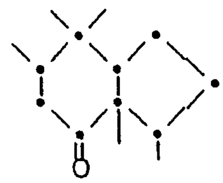

Analog Beispiel 2.

| | |
|---|---|
| Sdp.: | 76° C/0,02 Torr |
| Geruch: | camphrig, zuckrig, holzig, cedrig |
| MS: m/e = | 208(13), 193(100), 190(6), 175(10), 166(28), 153(62), 138(45), 123(93), 109-(90), 95(75), 81(50), 69(70), 55(55), 41(64) |
| IR (Film): | 1700, 1450, 1410, 1390, 1372, 1320, 1270, 1250, 1210, 1130, 1105, 1032, 945, 864 $cm^{-1}$ |
| NMR (400 MHz, $CDCl_3$): | 2 Isomere ca. 50/50: $\delta$ = 1,215 und 1,035 ppm (je 3H,s); $\delta$ = 0,995 und 0,94 ppm (je 3H,d,J=6,5 Hz und 7 Hz); $\delta$ = 0,905 und 0,90 ppm (je 3H,d,J=6,5 Hz und 7 Hz); $\delta$ = 0,935 und 0,895 ppm (je 3H,s); $\delta$ = 0,635 und 0,58 ppm (je 3H,s). |

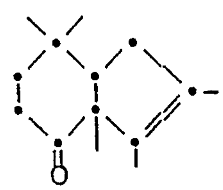

Vgl. Beispiel 3B.

| | |
|---|---|
| Sdp.: | 89° C/0,08 Torr |
| Geruch: | camphrig, minzig, nach Tannennadeln, holzig, leicht fruchtig - blumig |
| MS m/e: | 206(46), 191(12), 198(2), 163(20), 149(100), 135(25), 121(24), 108(80), 93-(69), 81(20), 77(24), 71(29), 55(25), 43(75) |
| IR (Film): | 1700, 1620, 1470, 1445, 1390, 1370, 1240, 1155, 1128, 1086, 1030/1020, 900, 870 $cm^{-1}$ |
| NMR (400 MHz, $CDCl_3$): | $\delta$ = 1,64 ppm (3H, schmalstes d,J= 1 Hz); $\delta$ = 1,48 ppm (1H, feinst aufgespaltenes m); $\delta$ = 1,415 ppm (3H, schmales m); $\delta$ = 1,21 ppm (3H,s); $\delta$ = 0,98 ppm (3H,s); $\delta$ = 0,92 ppm (3H,s). |

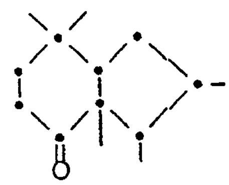

Analog Beispiel 2.

| | |
|---|---|
| Sdp.: | 90°C/0,1 Torr |
| Geruch: | camphrig, homogen, erdig, nach Terpentin, an Tannenharz erinnernd, holzig |
| MS: m/e = | 208(10), 193(30), 177(4), 175(5), 165(1), 152(33), 139(100), 137(31), 123(30), 109(60), 97(44), 83(37), 69(43), 55(64), 41(69) |
| IR (Film): | 1695, 1460, 1410, 1390/1380/1370, 1320, 1255, 1240, 1185, 1130, 1110, 1030, 890 $cm^{-1}$ |
| NMR (400 MHz, $CDCl_3$): | $\delta$ = 2,41 ppm (1H,dxdd,J=20/10/8 Hz); $\delta$ = 1,93 ppm (1H,dxdd,J=14/10/8 Hz); $\delta$ = 1,26 ppm (3H,s); $\delta$ = 0,94 ppm (3H,d,J=6,5 Hz); $\delta$ = 0,93 ppm (3H,s); $\delta$ = 0,87 ppm (3H,s); $\delta$ = 0,75 ppm (3H,d,J=7 Hz). |

Beispiel 4

Zu einer Lösung von 50 g Dihydro-α-ionon-epoxid in 1,5 l Methylenchlorid werden unter intensivem Rühren bei -25°C in 1-2 Minuten 5 ml Bortrifluoridätherat zugetropft. Dann wird das Reaktionsgefäss mittels Eisbad gekühlt. In ca. 15 Minuten erreicht das Reaktionsgemisch 0°C. Darauf wird mit 150 ml 2n Sodalösung 5 Minuten verrührt, die organische Phase abgetrennt und die wässrige Phase zweimal nachextrahiert. Die vereinigten Methylenchloridextrakte werden neutral gewaschen, getrocknet und bei 50°C am Wasserstrahlvakuum eingedampft. Vom Rohprodukt wird bei 50-68°C/0,035 Torr die Kopffraktion (6,5 g) abgetrennt, die flüchtige Nebenprodukte beinhaltet. Das Hauptprodukt II (41 g) destilliert bei 70-78°C/0,035 Torr. Die Analyse zeigt einen Gehalt von 80% 1-Formyl-1,3,3-trimethyl-2-[3-oxobutyl]-cyclopentan:

| | |
|---|---|
| MS: m/e = | 195(3), 192(5), 177(5), 167(14), 163(5), 154(17), 152(8), 149(15), 135(8), 123-(36), 109(46), 95(38), 81(24), 69(30), 58(18), 55(29), 43(100) |
| IR (Film): | 2710, 1720, 1460, 1368, 1245, 1160, 950 $cm^{-1}$ |
| NMR (400 MHz, $CDCl_3$): | $\delta$ = 9,70 ppm (1H,s); $\delta$ = 2,12 ppm (3H,s); $\delta$ = 1,175 ppm (3H,s); $\delta$ = 1,055 ppm (3H,s); $\delta$ = 0,89 ppm (3H,s). |

Dieses Material wird ohne weitere Reinigung cyclisiert. Dazu werden die 41 g Produkt in 410 ml methanolischer Kalilauge (1n KOH in 90% MeOH) während 50 Minuten auf Rückflusstemperatur erhitzt, abgekühlt, mit 3,5 l Wasser verdünnt und mit Hexan erschöpfend extrahiert. Nach dem üblichen Neutralwaschen, Trocknen und Abdampfen der organischen Phase im Wasserstrahlvakuum wird das ölige Rohprodukt fraktioniert destilliert. Nach den Kopffraktionen (4 g) geht das reine I (n=0), d.h. 3-Acetyl-1,6,6-trimethyl-bicyclo[3.3.0]-oct-2-en (22 g) bei 65°C/0,05 Torr über.

| | |
|---|---|
| UV (AetOH): | $\lambda_{max}$ = 240 nm ($\epsilon$=11800) |
| Geruch: | camphrig, fruchtig, holzig, Richtung p-tert. Butyl-cyclohexylacetat. |
| MS: m/e = | 192(5), 177(5), 159(3), 149(7), 136(7), 123(30), 107(15), 95(30), 79(9), 69(10), 55(10), 43(100) |
| IR (Film): | 1668, 1625/1612, 1455, 1372, 1362, 1305, 1265, 1230, 1170, 1042, 995, 985, 950, 868 $cm^{-1}$ |
| NMR (400 MHz, $CDCl_3$): | $\delta$ = 6,39 ppm (1H, schmales t, J=1 Hz); $\delta$ = 2,29 ppm (3H,s); $\delta$ = 1,86 ppm (1H,dd,J=8/4 Hz); $\delta$ = 1,235 ppm (3H,s); $\delta$ = 1,00 ppm (3H,s); $\delta$ = 0,85 ppm (3H,s). |

Beispiel 5

3,8 g ungesättigter Verbindung I (n=0) des Beispiels 4 werden in 50 ml Aethanol gelöst, dazu 500 mg KOH (85%ige Pillen) und dann 500 mg 5% Palladiumkohle gegeben und unter Wasserstoffatmosphäre 12 Stunden intensiv gerührt. Dann wird die Lösung konentriert, mit Hexan/Wasser verdünnt, vom Katalysator

abfiltriert und die organische Phase neutral gewaschen, getrocknet und eingedampft. Die resultierenden 3,5 g der hydrierten Verbindung I werden unter dem Vakuum einer Oelpumpe kurzwegdestilliert. Das reine Produkt, das 3-Acetyl-1,6,6-trimethyl-bicyclo-[3.3.0]-octan (2,9 g) zeigt einen camphrigen, blumigen, iononig-holzigen Geruch.

| | |
|---|---|
| MS: m/e = | 194(7), 179(10), 176(6), 161(9), 151(8), 136(5), 125(38), 109(60), 95(80), 81-(68), 69/68/67(23/20/21), 55(25), 43(100) |
| IR (Film): | 1712, 1460, 1385/1370/1360, 1275, 1218, 1168, 1120/1110, 1085, 1040, 1000, 960/947, 925, 910, 872, 850, 812 $cm^{-1}$ |
| NMR (400 MHz, $CDCl_3$): | Hauptisomer $\delta$ = 2,75 ppm (1H,dq, J=18/6 Hz); $\delta$ = 2,14 ppm (3H,s); $\delta$ = 1,15 ppm (3H,s); $\delta$ = 0,955 ppm (3H,s); $\delta$ = 0,93 ppm (3H,s). |

Analog Beispiel 4 und 5 werden folgende Verbindungen hergestellt :

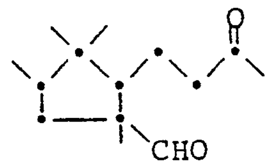

| | |
|---|---|
| MS: m/e = | 224(<1), 206(1), 196(1), 191(<1), 181(8), 167(5), 154(8), 137(13), 123(12), 109(11), 96(8), 95(10), 81(13), 69(19), 58(6), 55(13), 43(100) |
| IR (Film): | 2715, 1720, 1460, 1415, 1370, 1270, 1225, 1170, 1100, 1075/1060, 1025, 975, 950, 912, 870/855 $cm^{-1}$ |
| NMR (400 MHz, $CDCl_3$): | $\delta$ = 9,69 ppm (1H,s); $\delta$ = 2,485 ppm (2H,m); $\delta$ = 2,23 ppm (1H,dd,J=13/7 Hz); $\delta$ = 2,13 ppm (3H,s); $\delta$ = 1,84 ppm (1H, sextett, J=7 Hz); $\delta$ = 1,205 ppm (3H,s); $\delta$ = 1,17 ppm (1H,dd,J=13/7 Hz); $\delta$ = 0,94 ppm (3H,d,J=7 Hz); $\delta$ = 0,91 ppm u. 0,915 ppm (je 3H,s). |

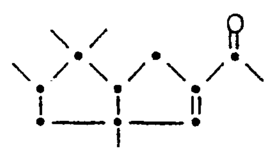

| | |
|---|---|
| Sdp.: | 75 °C/0,05 Torr |
| Geruch: | fruchtig, tabakartig, rosig, holzig, frisch, nach Eukalyptus |
| UV (AetOH): | $\lambda_{max}$ = 240 nm ($\epsilon$ = 12250) |
| MS: m/E = | 224(Spur), 206(10), 191(7), 173(3), 163(10), 149(5), 135(15), 123(83), 107-(25), 91(12), 77(11), 69(14), 55(16), 43(100) |
| IR (Film) | 1670, 1630, 1455, 1380, 1365, 1300, 1280, 1260, 1245, 1220, 1165, 1045, 1022, 960, 872 $cm^{-1}$ |
| NMR (400 MHz, $CDCl_3$): | $\delta$ = 6,32 ppm (1H,t,J=2 Hz); $\delta$ = 2,62 ppm (1H,ddxdd,J=17/9/2 Hz) und $\delta$ = 2,53 ppm (1H,ddxdd,J=17/4,5/2 Hz); $\delta$ = 2,285 ppm (3hH,s); $\delta$ = 2,00 ppm (1H,dd,J=9/4,5 Hz); $\delta$ = 1,745 ppm (1H,dd,J=13/6 Hz); $\delta$ = 1,56 ppm (1H, pseudo septett); $\delta$ = 1,38 ppm (1H,dd,J=12/11 Hz); $\delta$ = 1,27 ppm (3H,s); $\delta$ = 0,89 ppm (3H,s); $\delta$ = 0,845 ppm (3H,s); $\delta$ = 0,815 ppm (3H,d,J=6,5 Hz). |

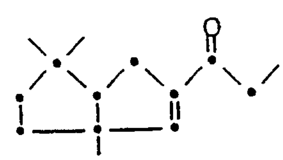

| | |
|---|---|
| UV (AetOH): | $\lambda_{max}$ = 240 nm ($\epsilon$=11200) |

| | |
|---|---|
| Sdp.: | 80° C/0,07 Torr |
| Geruch: | Camphrig, heiss, holzig, wurzelig, vetiverartig, nach Piniennadeln, grün, eukalyptusartig |
| MS: m/e = | 206(24), 191(6), 177(100), 159(13), 149(30), 137(28), 121(40), 107(49), 93-(32), 79/77(24), 69(29), 57(100), 41(25), 29(49) |
| IR (Film): | 1670, 1640/1615, 1460, 1415, 1375, 1365, 1300, 1250, 1225, 1210/1200, 1160, 1082, 1062, 1010/995/985, 930, 895, 875, 835, 800, 700 $cm^{-1}$ |
| NMR (400 MHz, $CDCl_3$): | $\delta$ = 6,38 ppm (1H,t,J=2 Hz); $\delta$ = 2,66 ppm (2H,q,J=7 Hz); $\delta$ = 2,56 und 2,505 ppm (je 1H,ddxdd); $\delta$ = 1,85 ppm (1H,dd,J=8/3,5 Hz); $\delta$ = 1,675 ppm (2H,m); $\delta$ = 1,395 ppm (2H,m); $\delta$ = 1,225 ppm (3H,s); $\delta$ = 1,095 ppm (3H,t,J=7 Hz); $\delta$ = 1,00 ppm (3H,s); $\delta$ = 0,85 ppm (3H,s). |

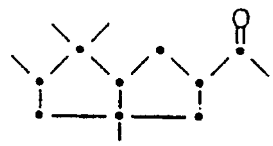

| | |
|---|---|
| Sdp.: | 90° C/0,08 Torr (Isomerengemisch) |
| Geruch: | Camphrig, holzig, tabakartig, patchouliartig, animalisch |
| MS: m/e = | 208(18), 193(12), 175(34), 165(5), 151(4), 135(25), 123(83), 109(37), 95(29), 81(100), 69(45), 55(34), 43(95) |
| IR (Film): | 1710, 1460, 1385/1375/1362, 1275, 1255/1245, 1235, 1215, 1165, 1100, 1045, 950 $cm^{-1}$ |
| NMR (400 MHz, $CDCl_3$): | $\delta$ = 2,835 ppm und $\delta$ = 2,725 ppm und $\delta$ = 2,695 ppm (zus. 1H,q,J=7 u. 6 u. 5 Hz); $\delta$ = 2,145 und 2,135 ppm (zus. 3H,s); $\delta$ = 1,13 u. 1,115 ppm (zus. 3H,s); $\delta$ = 0,805 ppm (3H,d,J=6 Hz); $\delta$ = 0,88 und 0,85 ppm (zus. 3H,s); $\delta$ = 0,78 und 0,77 ppm (zus. 3H,s). |

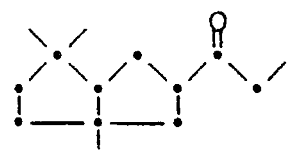

| | |
|---|---|
| Sdp.: | 90° C/0,08 Torr (2 Isomeren) |
| Geruch: | Balsamisch, fruchtig, patchouliartig, camphrig, holzig, leicht citrusartig |
| MS: m/e = | 208(8), 193(2), 179(27), 151(40), 139(6), 124(27), 109(39), 95(100), 81(77), 69(43), 57(61), 41(48), 29(54) |
| IR (Film): | 1710, 1455, 1410, 1385/1372/1360, 1335, 1118, 1040, 1025, 990, 958, 910, 880, 830, 795 $cm^{-1}$ |
| NMR (400 MHz, $CDCl_3$): | Hauptisomer: $\delta$ = 2,76 ppm (1H,m); $\delta$ = 2,455 ppm (2H,q,J=7 Hz); $\delta$ = 1,15 ppm (3H,s); $\delta$ = 1,04 ppm (3H,t,J=7 Hz); $\delta$ = 0,95 ppm (3H,s); $\delta$ = 0,92 ppm (3H,s). |

In den folgenden Beispielen bedeuten:

| | |
|---|---|
| Verbindung A: | 1,4,4,7a-Tetramethyl-3a,4,5,7a-tetahydro-7(6H)-ind-1-enon |
| Verbindung B: | 1-Aethyl-4,4,7a-trimethyl-3a,4,5,7a-tetrahydro-7(6H)-ind-1-enon |
| Verbindung C: | 3-Propionyl-1,6,6-trimethyl-bicyclo[3.3.0]-oct-2-en |
| Verbindung D: | 3-Acetyl-1,6,6,7-tetramethyl-bicyclo[3.3.0]-oct-2-en |
| Verbindung E: | 1,4,4,5,7a-Pentamethyl-3a,4,5,7a-tetrahydro-7(6H)-ind-1-enon |

## Beispiel 6

a) Allgemein blumige Base

| | Gewichtsteile |
|---|---|
| Terpineol | 260 |
| Hydroxycitronellal | 220 |
| Zimtalkohol-substitut | 120 |
| Phenyläthylalkohol | 100 |
| Cinnamylformiat | 20 |
| Linalool | 15 |
| Terpenylacetat | 10 |
| Keton-moschus | 10 |
| Geranylacetat | 10 |
| Jasmin synth. | 10 |
| Eugenol | 5 |
| Indol 10% DPG (Dipropylenglykol) | 5 |
| C-10-Aldehyd 10% DPG | 5 |
| p-Methylacetophenon | 5 |
| Undecalacton | 5 |
| DPG | 100 |
| | 900 |

Zugabe von 5% B

| | |
|---|---|
| Frisch getaucht: | Verstärkt, verbreitert und harmonisiert die Komposition, erzeugt grosse Frische. |
| Fond: | dito |

Zugabe von 5% C

| | |
|---|---|
| Frisch getaucht: | Macht die Komposition reicher, schwerer und süsser. |
| Fond: | Behält den süssen Charakter bis zum Schluss. |

Zugabe von 5% D

| | |
|---|---|
| Frisch getaucht: | Erzeugt grosse Strahlungskraft, verleiht der Komposition eine würzig-pfeffrige Note. |
| Fond: | dito |

b) Fruchtige Base

| | Gewichtsteile |
|---|---|
| Methyl-phenyl-glycidsäureäthylester | 50 |
| Aethyl-acetylacetat | 15 |
| Dimethyl-benzylbutyrat | 15 |
| Maltylisobutyrat | 10 |
| Benzylacetat | 5 |
| Aethylacetat | 5 |
| DPG | 795 |
| | 900 |

Zugabe von 5% D

Frisch getaucht: Verstärkt die Süssigkeit der Komposition und vermittelt angenehmen Waldduft.

Zugabe von 5% B

Frisch getaucht: Verleiht der Komposition eine wunderbare, süsse Honig-Note.

Zugabe von 5% C

Frisch getaucht: Verleiht der Komposition eine frische, leicht holzige (Fichtennadel)-Note.
Alle drei erzielen im Fond: Frische-Effekt.

c) Rosen-Base

| | Gewichtsteile |
|---|---|
| Phenyläthylalkohol | 300 |
| Geraniol | 250 |
| Jasmin "lavage" | 200 |
| Citronellol | 100 |
| α-Jonon | 40 |
| C-10-Aldehyd 10% DPG | 5 |
| C-11-Aldehyd 10% DPG | 5 |
| | 900 |

Zugabe von 5% C

Frisch getaucht: Harmonisiert, komplettiert den natürlichen Rosenduft. Erinnert nun an die frisch geöffnete Knospe einer Gartenrose.

Fond: Harmonisiert und verstärkt den Fond; homogene Wirkung bis zum Schluss.

Zugabe von 5% D

| | |
|---|---|
| Frisch getaucht: | Siehe oben. Erinnert jedoch an eine edle Zuchtrose mit dem grünen Aspekt von Blattwerk. |
| Fond: | Siehe oben. |

d) Fougère-Base

| | Gewichtsteile |
|---|---|
| Lavendelöl franz. | 200 |
| Linalylacetat | 150 |
| Baum-Moos | 60 |
| Coumarin | 50 |
| Patchouliöl | 30 |
| Rhodinol ex Geraniumöl | 30 |
| Methyldihydrojasmonat | 30 |
| Keton-moschus | 30 |
| Vetivenylacetat | 30 |
| Geraniumöl-substitut | 30 |
| Amylsalicylat | 20 |
| Sandela | 20 |
| Linalool | 20 |
| Benzylacetat | 15 |
| Ylang-Ylangöl | 15 |
| Eugenol | 15 |
| Thymianöl | 5 |
| DPG | 50 |
| | 800 |

Zugabe von 5% E

| | |
|---|---|
| Frisch getaucht: | Verfeinert die Komposition, rundet sie ab und nimmt ihr die Härte. |
| Fond: | Macht die Komposition reicher, feiner und weicher. |

Zugabe von 5% B

| | |
|---|---|
| Frisch getaucht: | Verstärkt den krautigen Aspekt der Base. |
| Fond: | Wirkt trocken, tabakartig. |

e) <u>Chypre-Base</u>

| | Gewichtsteile |
|---|---|
| Madrox ® (1-Methylcyclododecyl-methyläther) | 200 |
| Bergamotteöl | 150 |
| Hydroxycitronellal | 100 |
| Fichtennadelöl | 80 |
| Citronellol | 80 |
| Petitgrainöl | 60 |
| Corianderöl | 40 |
| Galbanumöl | 40 |
| Zedernholzöl | 40 |
| Patchouliöl | 40 |
| Zitronenöl | 40 |
| Eichenmoos entfärbt | 20 |
| Elemiöl | 10 |
| DPG | 60 |
| | 960 |

Zugabe von 5% E

Frisch getaucht: Verfeinert die frische Note der Komposition und umgibt sie mit einer angenehmen Kühle.
Fond: Verfeinert die ganze Komposition.

Zugabe von 5% A

Frisch getaucht: Siehe oben.
Fond: Verstärkt und verfeinert die Eichenmoos-Note der Komposition.

f) Fichten-Base

| | Gewichtsteile |
|---|---|
| Rosmarin-Essenz | 200 |
| Bornyl-acetat | 200 |
| Linalool | 200 |
| Terpineol | 100 |
| Vertofix coeur | 100 |
| Galaxolid 50% | 50 |
| Sapin ess. Aiguilles (Tannennadel-Essenz) | 50 |
| Anhydrol Fir Balsam (Tannenharz-Anhydrol) | 30 |
| Eucalyptol | 25 |
| Borneol krist. | 10 |
| Salbeiöl jugoslawisch | 10 |
| C-12-Aldehyd (MNA) 10% DPG | 5 |
| C-10-Aldehyd 10% DPG | 5 |
| | 995 |

Zugabe von 5% A

Frisch getaucht: Kopfnote frischer, stärker, kräftiger und strahlender.
Fond: Die Haftfestigkeit wird eindeutig verbessert und verstärkt.

Zugabe von 5% E

Frisch getaucht: Wie oben.
Fond: Wie oben.
Zugabe von 5% B, Frisch: Wie oben. Fond: Wie oben.

g) Tabak-Base

| | Gewichtsteile |
|---|---|
| o-tert-Butylcyclohexylacetat | 400 |
| Jasminöl synth. | 300 |
| Keton-moschus | 40 |
| Sandela | 40 |
| Styrallylacetat | 30 |
| Coumarin | 20 |
| Isobutylchinolin 10% DPG | 10 |
| Lavendelöl | 10 |
| Vetiveröl | 10 |
| Galbanumöl | 10 |
| Vassuraöl | 10 |
| DPG | 40 |
| | 920 |

Zugabe von 5% A

Frisch getaucht: Verstärkt und rundet die blumige Note ab, verleiht der Komposition grosse Strahlungskraft, wirkt frischer.
Fond: Kräftiger.

Zugabe von 5% E

Frisch getaucht: Veredelt die krautig-holzige Note der Komposition.
Fond: Wie oben.

Zugabe von 5% B

Frisch getaucht: Wirkt auf die Kompositionsbase harmonisierend.
Fond: Wie oben.

h) Tabakaroma

Ein Tabakaroma (sog. top flavour, Typ Virginia) kann wie folgt zusammengesetzt sein:

| | Gewichtsteile | |
|---|---|---|
| Thibetolid | 4 | 4 |
| Sandelholzöl | 15 | 15 |
| Essigsäure-cedrylester | 15 | 15 |
| α-Jonon | 1 | 1 |
| ß-Jonon | 5 | 5 |
| Keto-isophoron | 20 | 15 |
| ß-Caryophyllen | 5 | 5 |
| Essigsäure-geranylester | 5 | 5 |
| Propylenglykol | 400 | 400 |
| Alkohol 96% | 530 | 530 |
| A | --- | 5 |
| | 1'000 | 1'000 |

Durch den Zusatz von A wird in obiger Komposition die ursprünglich vorhandene, süssliche, holzige Note deutlich reduziert. Beim Abrauchen des aromatisierten Tabaks tritt eine angenehme tabakartige, teerartig/holzige Note hervor, die an Virginia-Tabak erinnert.

**Patentansprüche**

1. Verbindungen der allgemeinen Formel

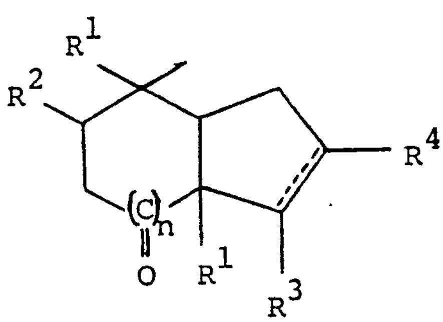

I

worin $R^1$ Methyl oder Aethyl, $R^2$ Wasserstoff oder Methyl, und falls n = 0, $R^3$ = Wasserstoff und $R^4$ Acetyl oder Propionyl, und, falls n = 1, $R^3$ = Methyl oder Aethyl und $R^4$ Wasserstoff oder Methyl darstellen, und die gestrichelte Linie eine fakultative C-C-Bindung darstellt,
mit der Ausnahme von 1,4,4,7a-Tetramethyl-3a,4,5,7a-tetrahydro-7(6H)-ind-1-enon (Ia).

2. Verbindungen der Formel I gemäss Anspruch 1, worin n = 0.

3. Verbindungen der Formel I gemäss Anspruch 1, worin n = 1, mit der Ausnahme von 1,4,4,7a-Tetramethyl-3a,4,5,7a-tetrahydro-7(6H)-ind-1-enon (Ia).

4. 1,4,4,5,7a-Pentamethyl-3a,4,5,7a-tetrahydro-7(6H)-ind-1-enon.

5. 1-Aethyl-4,4,7a-trimethyl-3a,4,5,7a-tetrahydro-7(6H)-ind-1-enon.

6. 1,2,4,4,7a-Pentamethyl-3a,4,5,7a-tetrahydro-7(6H)-ind-1-enon.

7. 3-Acetyl-1,6,6-trimethyl-bicyclo-[3.3.0]-oct-2-en.

8. 3-Acetyl-1,6,6,7-tetramethyl-bicyclo-[3.3.0]-oct-2-en.

9. 3-Propionyl-1,6,6-trimethyl-bicyclo-[3.3.0]-oct-2-en.

10. Riech- und/oder Geschmackstoffkomposition, gekennzeichnet durch einen Gehalt an einer Verbindung der Formel

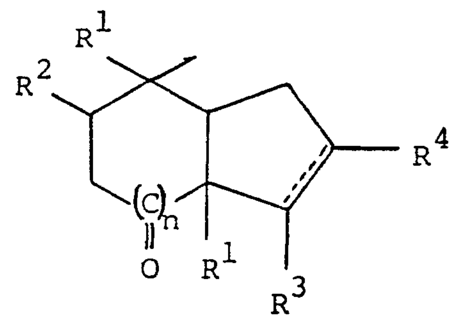

I

worin $R^1$ Methyl oder Aethyl, $R^2$ Wasserstoff oder Methyl, und falls n = 0, $R^3$ = Wasserstoff und $R^4$ Acetyl oder Propionyl, und, falls n = 1, $R^3$ = Methyl oder Aethyl und $R^4$ Wasserstoff oder Methyl darstellen, und die gestrichelte Linie eine fakultative C-C-Bindung darstellt.

11. Verwendung der Verbindungen der allgemeinen Formel

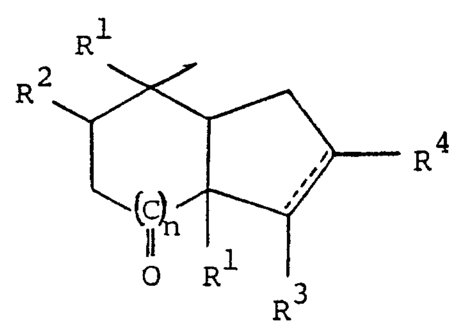

I

worin $R^1$ Methyl oder Aethyl, $R^2$ Wasserstoff oder Methyl, und falls n = 0, $R^3$ = Wasserstoff und $R^4$ Acetyl oder Propionyl, und, falls n = 1, $R^3$ = Methyl oder Aethyl und $R^4$ Wasserstoff oder Methyl darstellen, und die gestrichelte Linie eine fakultative C-C-Bindung darstellt,
als Riech- und/oder Geschmackstoffe.

12. Verwendung von 1,4,4,7a-Tetramethyl-3a,4,5,7a-tetrahydro-7(6H)-ind-1-enon, 1,4,4,5,7a-Pentamethyl-3a,4,5,7a-tetrahydro-7(6H)-ind-1-enon, 1-Aethyl-4,4,7a-trimethyl-3a,4,5,7a-tetrahydro-7(6H)-ind-1-enon, 1,2,4,4,7a-Pentamethyl-3a,4,5,7a-tetrahydro-7(6H)-ind-1-enon, 3-Acetyl-1,6,6-trimethyl-bicyclo-[3.3.0]-oct-2-en, 3-Acetyl-1,6,6,7-tetramethyl-bicyclo-[3.3.0]-oct-2-en oder 3-Propionyl-1,6,6-trimethyl-bicyclo-[3.3.0]-oct-2-en als Riech- und/oder Geschmackstoff.

13. Verfahren zur Herstellung der Verbindungen der allgemeinen Formel

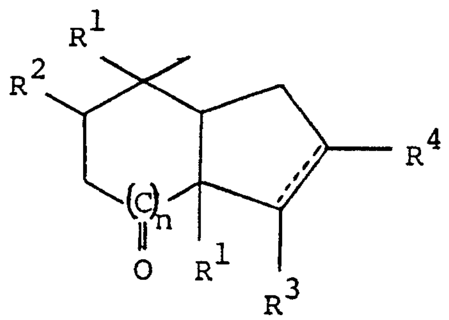

I

worin $R^1$ Methyl oder Aethyl, $R^2$ Wasserstoff oder Methyl, und falls n = 0, $R^3$ = Wasserstoff und $R^4$ Acetyl oder Propionyl, und, falls n = 1, $R^3$ = Methyl oder Aethyl und $R^4$ Wasserstoff oder Methyl darstellen, und die gestrichelte Linie eine fakultative C-C-Bindung darstellt,
dadurch gekennzeichnet, dass man eine Verbindung der Formeln

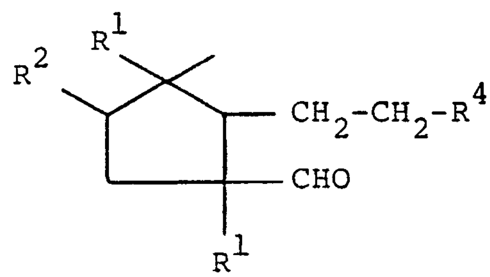

II

worin $R^1$ und $R^2$ obige Bedeutungen besitzen und $R^4$ Acetyl oder Propionyl bedeutet,
oder

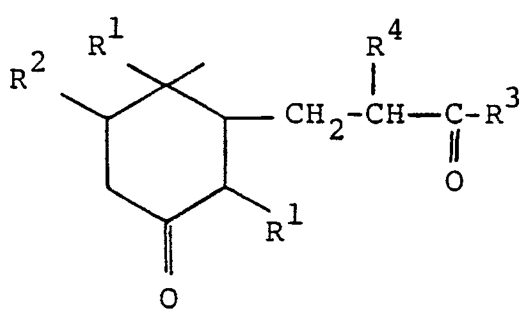

III

worin $R^1$ und $R^2$ obige Bedeutung besitzen, $R^3$ Methyl oder Aethyl und $R^4$ Wasserstoff oder Methyl darstellen,
cyclisiert und, gegebenenfalls, die C=C-Doppelbindung des erhaltenen Reaktionsproduktes hydriert.

**14.** Verfahren nach Anspruch 13, dadurch gekennzeichnet, dass man von einer in situ hergestellten Verbindung der Formeln II oder III ausgeht.

## Claims

**1.** Compounds of the general formula

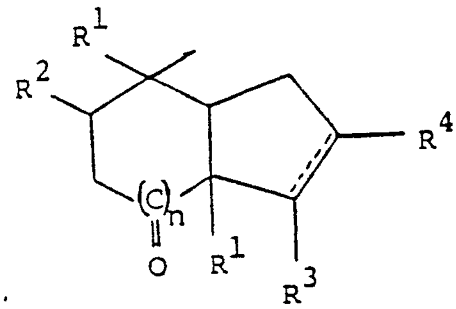

I

wherein $R^1$ represents methyl or ethyl, $R^2$ represents hydrogen or methyl and, where n = 0, $R^3$ = hydrogen and $R^4$ represents acetyl or propionyl and, where n = 1, $R^3$ = methyl or ethyl and $R^4$ represents hydrogen or methyl, and the dotted line represents an optional C-C bond, with the exception of 1,4,4,7a-tetramethyl-3a,4,5,7a-tetrahydro-7(6H)-ind-1-enone (Ia).

**2.** Compounds of formula I in accordance with claim 1, wherein n = 0.

**3.** Compounds of formula I in accordance with claim 1, wherein n = 1, with the exception of 1,4,4,7a-tetramethyl-3a,4,5,7a-tetrahydro-7(6H)-ind-1-enone (Ia).

**4.** 1,4,4,5,7a-Pentamethyl-3a,4,5,7a-tetrahydro-7(6H)-ind-1-enone.

**5.** 1-Ethyl-4,4,7a-trimethyl-3a,4,5,7a-tetrahydro-7(6H)-ind-1-enone.

**6.** 1,2,4,4,7a-Pentamethyl-3a,4,5,7a-tetrahydro-7(6H)-ind-1-enone.

**7.** 3-Acetyl-1,6,6-trimethyl-bicyclo-[3.3.0]-oct-2-ene.

**8.** 3-Acetyl-1,6,6,7-tetramethyl-bicyclo-[3.3.0]oct-2-ene.

**9.** 3-Propionyl-1,6,6-trimethyl-bicyclo-[3.3.0]-oct-2-ene.

**10.** An odorant and/or flavouring composition, characterized in that it contains a compound of the formula

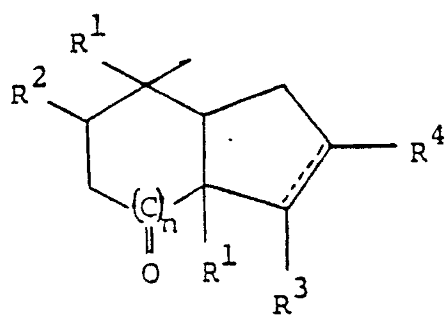

I

wherein $R^1$ represents methyl or ethyl, $R^2$ represents hydrogen or methyl and, where n = 0, $R^3$ = hydrogen and $R^4$ represents acetyl or propionyl and, where n = 1, $R^3$ = methyl or ethyl and $R^4$ represents hydrogen or methyl, and the dotted line represents an optional C-C bond.

**11.** The use of the compounds of the general formula

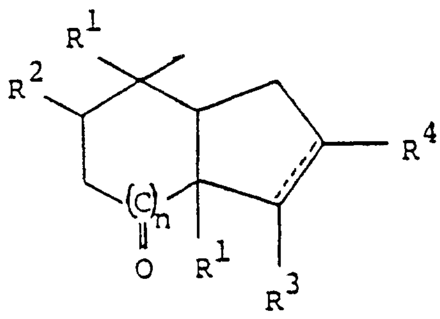

I

wherein $R^1$ represents methyl or ethyl, $R^2$ represents hydrogen or methyl and, where n = 0, $R^3$ = hydrogen and $R^4$ represents acetyl or propionyl and, where n = 1, $R^3$ = methyl or ethyl and $R^4$ represents hydrogen or methyl, and the dotted line represents an optional C-C bond, as odorants and/or flavorants.

12. The use of 1,4,4,7a-tetramethyl-3a,4,5,7a-tetrahydro-7(6H)-ind-1-enone, 1,4,4,5,7a-pentamethyl-3a,4,5,7a-tetrahydro-7(6H)-ind-1-enone, 1-ethyl-4,4,7a-trimethyl-3a,4,5,7a-tetrahydro-7(6H)-ind-1-enone, 1,2,4,4,7a-pentamethyl-3a,4,5,7a-tetrahydro-7(6H)-ind-1-enone, 3-acetyl-1,6,6-trimethyl-bicyclo-[3.3.0]-oct-2-ene, 3-acetyl-1,6,6,7-tetramethyl-bicyclo-[3.3.0]-oct-2-ene or 3-propionyl-1,6,6-trimethyl-bicyclo-[3.3.0]-oct-2-ene as an odorant and/or flavorant.

13. A process for the manufacture of the compounds of the general formula

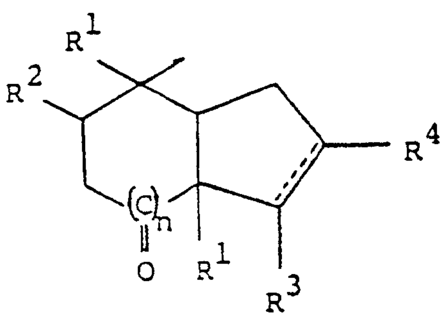

I

wherein $R^1$ represents methyl or ethyl, $R^2$ represents hydrogen or methyl and, where n = 0, $R^3$ = hydrogen and $R^4$ represents acetyl or propionyl and, where n = 1, $R^3$ = methyl or ethyl and $R^4$ represents hydrogen or methyl, and the dotted line represents an optional C-C bond, characterized by cyclizing a compound of the formula

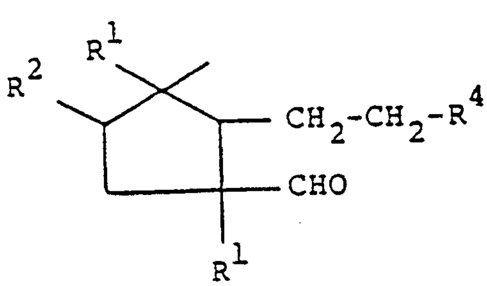

II

wherein $R^1$ and $R^2$ have the above significances and $R^4$ signifies acetyl or propionyl, or

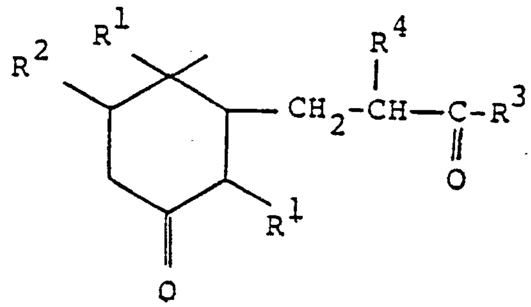

III

wherein $R^1$ and $R^2$ have the above significance, $R^3$ represents methyl or ethyl and $R^4$ represents hydrogen or methyl,
and, if desired, hydrogenating the C=C double bond present in the reaction product obtained.

14. A process according to claim 13, characterized in that the compound of formula II or III used as the starting material is prepared in situ.

**Revendications**

1. Composés de formule générale

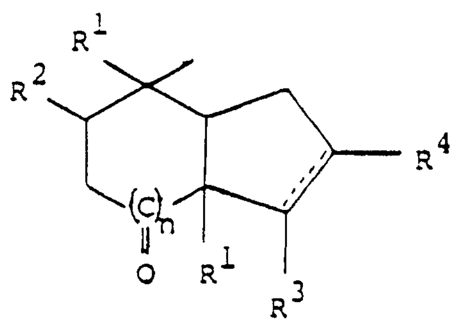

I

où $R^1$ représente un méthyle ou un éthyle, $R^2$ représente un hydrogène ou un méthyle, et, si n = 0, $R^3$ est un hydrogène et $R^4$ est un acétyle ou un propionyle et, si n = 1 $R^3$ est un méthyle ou un éthyle, et $R^4$ est un hydrogène ou un méthyle, et la ligne pointillée représente une liaison C-C facultative,
à l'exception de la 1,4,4,7a-tétraméthyl-3a,4,5,7a-tétrahydro-7(6H)-ind-1-énone (Ia).

2. Composés de formule I selon la revendication 1, où n = 0.

3. Composés de formule I selon la revendication I, où n = 1, à l'exception de la 1,4,4,7a-tétraméthyl-3a,4,5,7a-tétrahydro-7(6H)-ind-1-énone (Ia).

4. 1,4,4,5,7a-pentaméthyl-3a,4,5,7a-tétrahydro-7(6H)-ind-1-énone.

5. 1-éthyl-4,4,7a-triméthyl-3a,4,5,7a-tétrahydro-7(6H)-ind-1-énone.

6. 1,2,4,4,7a-pentaméthyl-3a,4,5,7a-tétrahydro-7(6H)-ind-1-énone.

7. 3-acétyl-1,6,6-triméthyl-bicyclo-[3.3.0]-oct-2-ène.

8. 3-acétyl-1,6,6,7-tétraméthyl-bicyclo-[3.3.0]-oct-2-ène.

9. 3-propionyl-1,6,6-triméthyl-bicyclo-[3.3.0]-oct-2-ène.

10. Compositions odorantes et/ou gustatives, caractérisées en ce qu'elles contiennent un composé de

formule

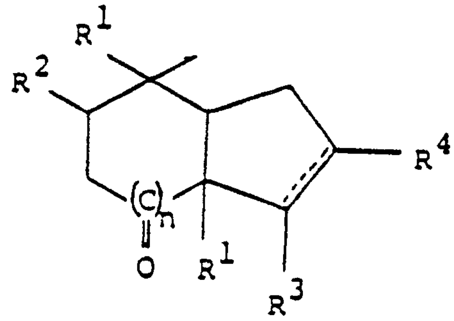

I·

où $R^1$ représente un méthyle ou un éthyle, $R^2$ un hydrogène ou un méthyle, et si n vaut 0, $R^3$ représente un hydrogène et $R^4$ représente un acétyle ou un propionyle et si n vaut 1, $R^3$ représente un méthyle ou un éthyle et $R^4$ représente un hydrogène ou un méthyle, et la ligne pointillée représente une liaison C-C supplémentaire.

**11.** Application des composés de formule générale

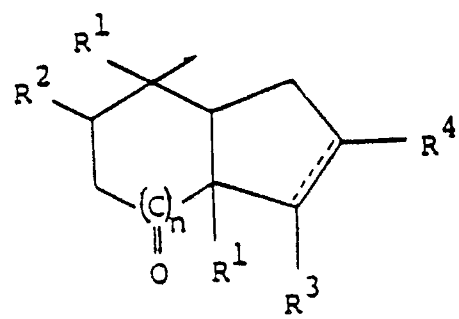

I

où $R^1$ représente un méthyle ou un éthyle, $R^2$ représente un hydrogène ou un méthyle, et si n vaut 0, $R^3$ représente un hydrogène et $R^4$ représente un acétyle ou un propionyle, et si n vaut 1, $R^3$ représente un méthyle ou un éthyle et $R^4$ représente un hydrogène ou un méthyle et la ligne pointillée représente une liaison C-C supplémentaire,
comme composés odorants et/ou gustatifs.

**12.** Application de la 1,4,4,7a-tétraméthyl-3a,4,5,7a-tétrahydro-7(6H)-ind-1-énone,
de la 1,4,4,5,7a-pentaméthyl-3a,4,5,7a-tétrahydro-7(6H)-ind-1-énone,
de la 1-éthyl-4,4,7a-triméthyl-3a,4,5,7a-tétrahydro-7(6H)-ind-1-énone,
de la 1,2,4,4,7a-pentaméthyl-3a,4,5,7a-tétrahydro-7(6H)-ind-1-énone,
du 3-acétyl-1,6,6-triméthyl-bicyclo-[3.3.0]-oct-2-ène,
du 3-acétyl-1,6,6,7-tétraméthyl-bicyclo-[3.3.0]-oct-2-ène,
ou du 3-propionyl-1,6,6-triméthyl-bicyclo-[3.3.0]-oct-2-ène comme composés odorants et/ou gustatifs.

**13.** Procédé de préparation des composés de formule générale

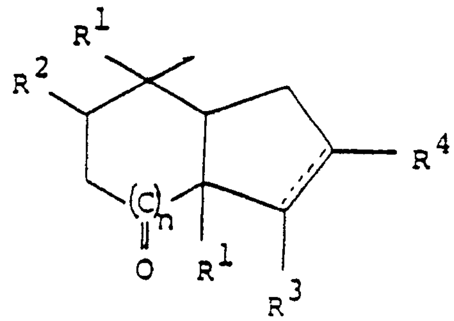

I

où $R^1$ représente un méthyle ou un éthyle, $R^2$ représente un hydrogène ou un méthyle, et si n vaut 0, $R^3$ représente un hydrogène et $R^4$ représente un acétyle ou un propionyle, et si n vaut 1, $R^3$ représente un méthyle ou un éthyle et $R^4$ représente un hydrogène ou un méthyle et la ligne pointillée représente une liaison C-C facultative,
caractérisé en ce qu'on cyclise un composé de formules

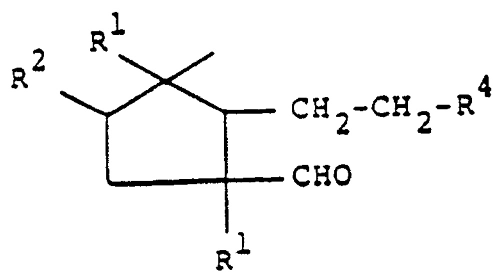

II

où $R^1$ et $R^2$ ont les significations données ci-dessus et $R^4$ représente un acétyle ou un propionyle,

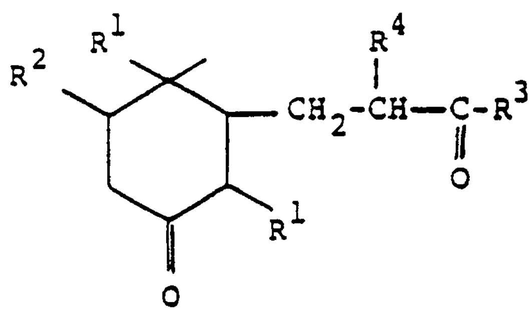

III

où $R^1$ et $R^2$ ont la signification donnée ci-dessus, $R^3$ représente un méthyle ou un éthyle et $R^4$ représente un hydrogène ou un méthyle,
et, le cas échéant, on hydrogène la double liaison C=C du produit réactionnel obtenu.

**14.** Procédé selon la revendication 13, caractérisé en ce qu'on part d'un composé de formules II ou III préparé in situ.